# EUROPEAN PATENT APPLICATION

(11) **EP 1 538 195 A1**
(43) Date of publication of application: **08.06.2005**
(21) Application number: 03257631.6
(22) Date of filing: 04.12.2003
(51) Int. Cl.: C12M 1/24, C12M 3/00, B01L 3/08

(54) **Cell culture device**

(71) Applicant: The Automation Partnership (Cambridge) Limited, Royston, Hertfordshire SG8 5WY (GB)
(72) Inventor: Malinge, David S., Royston, Hertfordshire SG8 9DE (GB)
(74) Representative: Brunner, Michael John

(57) **Abstract**

A cell culture device 1 comprises a flask 10 within which, in use, cells are cultured. The flask 10 is connected to a structure 100 that is constructed to enable the flask 10 to be held, in use, within a recess of a cell culture system. The volume of the envelope defined by the structure 100 and the flask 10 is greater than the volume of the flask 10.

## Description

The present invention relates to a cell culture device.

It is common, within the field of cell biology, to culture cells in order to harvest biologically active compounds produced by the cells, or indeed the cells themselves. Such cells are generally cultured on static plates which are enclosed in a bottle or flask. Flasks may generally be accessed through a neck portion, closed by means of a cap. When the flask is in use it is laid on its side so that the maximum possible surface area is horizontal. The cell cultivating medium covers the inner surface of this flask wall. Over time, the industry has developed a number of sizes of flask that are considered to be standard. One of these, known as the T-flask has four orthogonal walls (two major walls and two minor walls) and is configured so that the maximum surface area is available to the cells when the flask is laid on one of its major sides. These flasks are then stored in large automated storage systems consisting of a large number of recesses into which the flasks can be placed for storage or transport into, for example, an incubator. Cells are cultured within the flask within a cell culture medium that is isolated on the inner surface of the wall on which the flask rests. The flasks rest on a major wall in order to provide the maximum surface area.

It is well known within the field of cell biology that cell culture growth is optimised by providing the correct concentration of growing medium for the growing area that is defined by the major wall of the culture flask. However, it can be difficult to begin a cell culture in a standard T-flask as the concentrations may be too low as a result of the large surface area available. The industry is increasingly tending towards the use of smaller volumes and there is therefore a need to provide a smaller culture flask in which optimum growing conditions may be provided with a smaller quantity of growth medium. However, the problem arises as to how to store and transport these new smaller flasks as they do not fit conveniently in to pre-existing storage and incubation systems as these are configured to deal with standard flasks that have previously been developed by the industry, all of which are larger than the new generation of small flasks. However, the storage and incubation systems into which these flasks are usually placed are frequently large and complex devices that cannot easily or cheaply be replaced. The present invention therefore aims to provide a cell culture device that will enable a small growing area for cells to be isolated within a standard flask storage device.

According to the present invention there is provided a cell culture device comprising
a flask within which, in use, cells are cultured;
a structure connected to the flask and constructed to enable the flask to be held, in use, within a recess of a cell culture system;
wherein the volume of the envelope defined by the structure and the flask is greater than the volume of the flask.

The present invention therefore allows a small flask to be held securely within a pre-existing automated storage system provided with recesses considerably larger in size than the small flask to be stored. The principle is not limited to the storage of the small flasks but applies equally to any piece of automated laboratory equipment where flasks are held in position during a procedure, for example, during incubation in an incubator or low temperature storage in a freezer.

Preferably the flask is held within the envelope defined by the structure which may comprise a cuboid framework. This enables the structure to interface fully with the storage position within the automated storage system. This provides a greater level of security for the contents of the flask as the movement of the culture device within the storage device is reduced.

The structure may comprise a larger flask such as a T-flask or other industry standard flask. These flasks are readily available and are designed to fit within the automated lab systems and will therefore be exactly the right size. Furthermore, the enclosed space around the small flask in which the cells are cultured could be filled with a fluid or particulate solid in order to effect differing conditions from those in the surrounding flasks.

Alternatively, the larger flask may be empty so that, once the cell culture has reached its maximum population density within the small flask the contents may be transferred in to the larger flask using, for example, a pipette and culturing may continue within the larger flask. This allows any form of identification that has been applied to the flask to remain valid whilst the medium is cultured in firstly the smaller and latterly the larger flask.

The larger flask may further be provided with an opening that it arranged so as to permit access to the contents of the smaller flask. The neck portions of the smaller and larger flasks may be aligned to allow direct access using, for example, a pipette to extract the contents of the smaller flask. Alternatively, the small flask may be located off centre to a small degree provided that a pipette can still pass through the neck portion of the larger flask and access the contents of the smaller flask.

The smaller flask may further be provided with a closure member. Such a closure member maintains an enclosed environment within the smaller flask and ensures that, even if the flask is dropped, the contents remain safely within the smaller flask.

A spacing member may further be provided to separate the flask from the structure.

Alternatively, the small flask and the larger flask may share one wall.

Several examples of a cell culture device according to the present invention will now be described with reference to the accompanying drawings in which:
Figure 1 shows a plan view of a first example of the present invention,
Figure 2 shows a side view taken along the section A-A of Figure 1,
Figure 3 shows an end view taken along the section B-B of Figure 1,
Figure 4 shows a plan view of a second example of the present invention,
Figure 5 shows a side view taken along the section A-A of Figure 4,
Figure 6 shows an end view taken along the section B-B of Figure 4,
Figures 7A and 7B show plan and side views of a pipette accessing the contents of the first example of the present invention, and
Figures 8A, 8B and 8C show plan, side and perspective view of a part of an automated storage system comprising a number of cell culture devices according to the present invention.

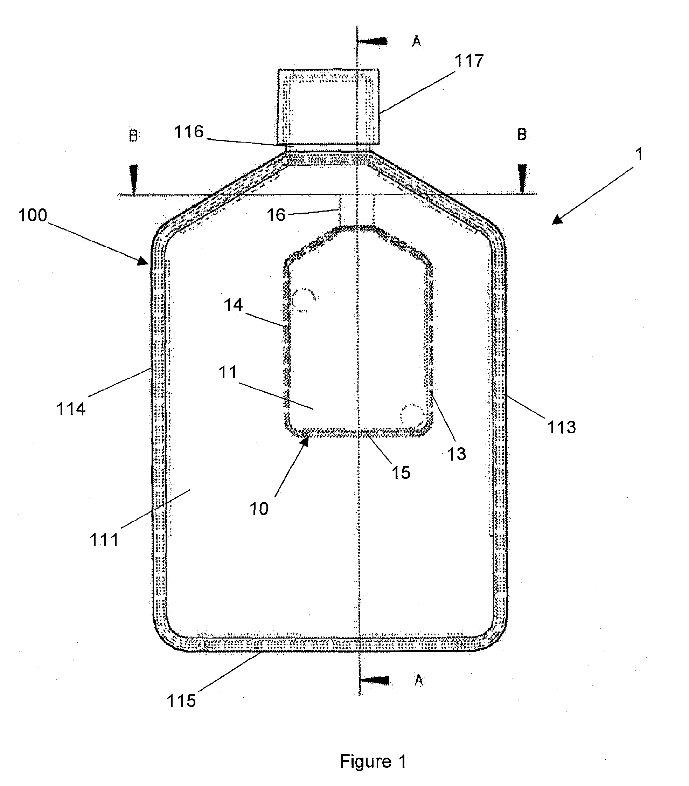

Figure 1 shows a first example of a cell culture device 1 according to the present invention. Within the internal volume of the culture device 1 there is provided a smaller flask 10 in which cells may be cultured. The flask 10 consists of are four orthogonal walls 11, 12, 13 and 14 and a base 15. There are two major walls 13 and 14 and two minor walls 11 and 12. Above the four orthogonal walls 11 to 14 the walls of the flask 10 converge to meet at a neck portion 16. The narrowing of the flask 10 at the neck portion 16 ensures that the contents of the flask 10 remain within the flask 10. The neck 16 could screw threaded and may co-operate, in use, with a cap 17 to seal the contents of the flask from the ambient atmosphere and also from the contents of the cell culture device 1. The use of a cap 17 to close the smaller flask 10 is only appropriate in some applications as a result of potential contamination of the sample held within the flask 10. In order to overcome this problem a septum seal (not shown) may be used to close the smaller flask 10. A septum seal is not removed but rather punctured to gain access to the contents of the inner flask 10 thereby reducing the danger of contamination of a sample.

Connected to the flask 10 is a structure 100 that forms the outer boundary of the culture device 1. In one example of the present invention the structure takes the form of a flask 100 of rectangular cross-section having four orthogonal walls 111, 112, 113, and 114 and a base 115 at one end. There are two major walls 113, 114 and two minor walls 111, 112. At the other end of the flask 100 the four orthogonal walls 111 to 114 the walls converge to meet at a neck portion 116. The neck 116 is screw threaded and co-operates in use, with a closure member, for example a cap 117 to seal the contents of the cell culture device 1 from the ambient atmosphere. The surface area of the major wall 11 of the small flask 10 is between 15% and 50% of the surface area of the major wall of the larger flask 100.

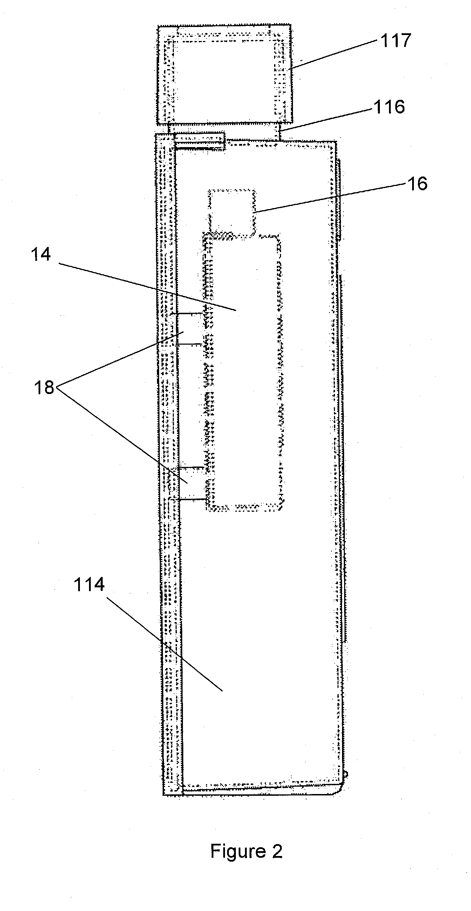

Figure 2 shows a side view of the first example of the culture device 1 according to the present invention. This shows the spacing member 18 which maintains the flask 10 in a stable position within the cell culture device 1.

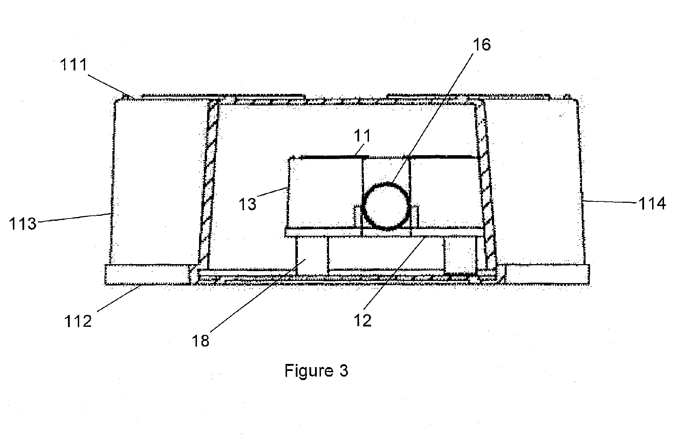

Figure 3 shows an end view of the cell culture device 1, partly in section. This shows that the neck portion 16 of the flask 10 and the neck portion 116 of the flask 100 are sufficiently aligned for access to be made to the flask 10 through the neck portion of the culture device 1.

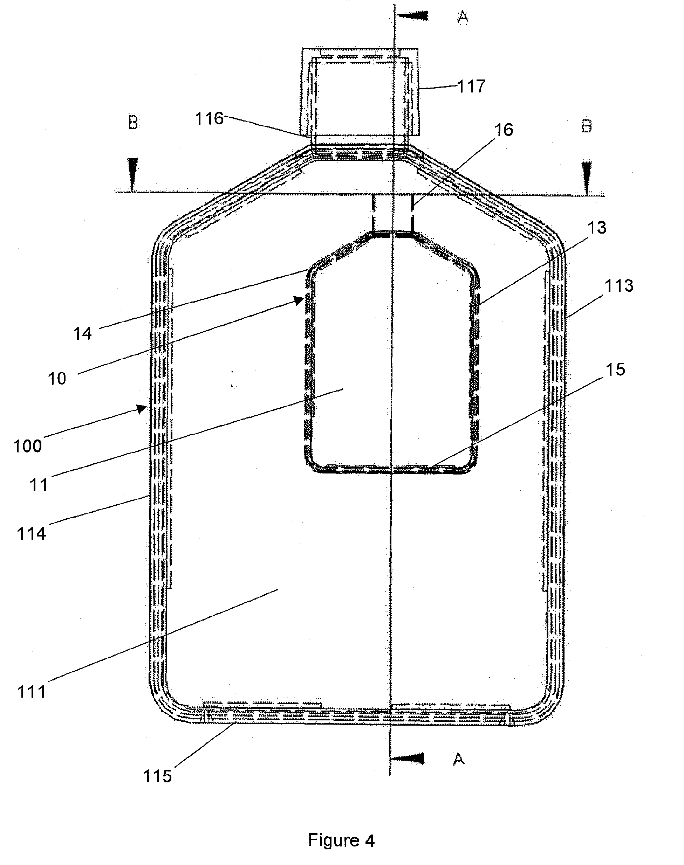

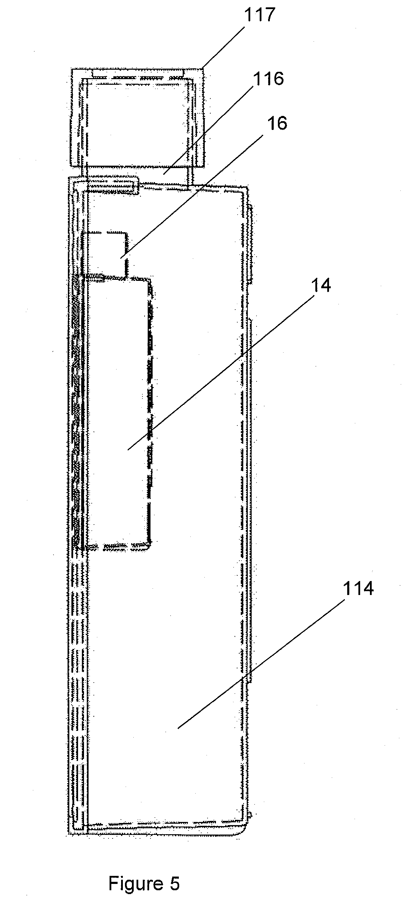

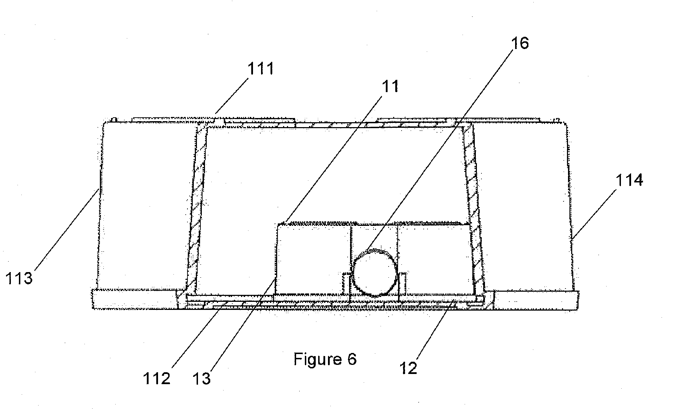

Figures 4 to 6 show a second example of a culture device 1 according to the present invention. Like reference numerals have been used throughout the description of these figures where appropriate. In this second example of a culture device 1 the flask 10 has exterior walls 11 and 14 and base portion 15 that also form part of the walls 111, 114 and base 115 of the cell culture device 1. Figure 5 shows a side view of the cell culture device 1 and Figure 6 shows an end view.

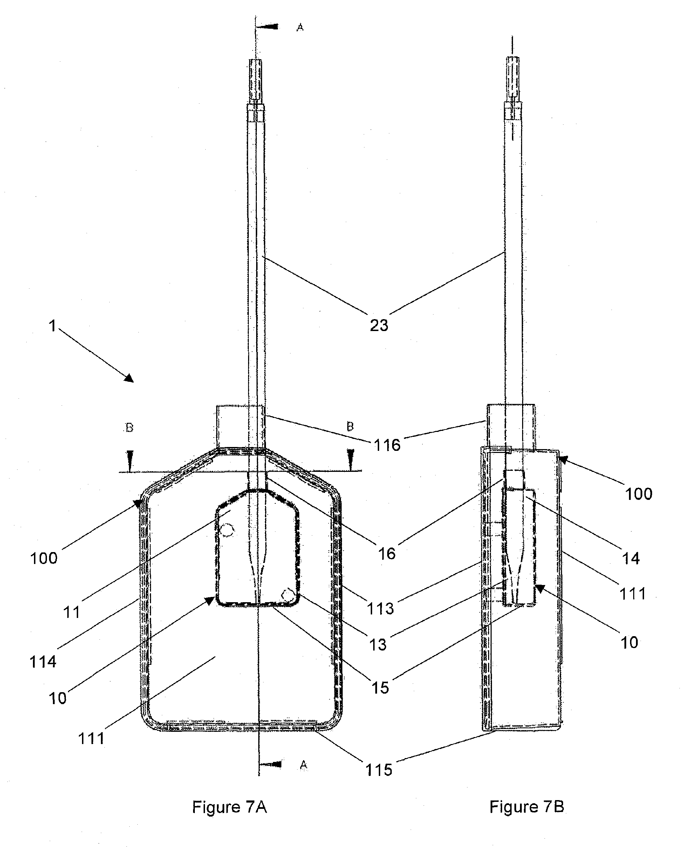

In use a suspension comprising the cells to be cultured and culture medium is introduced into the flask 10 using a pipette 23 as shown in Figure 7. The major wall 11 onto which the cells are to be cultured may be pre-treated with an agent that will assist the cells to adhere to the inner surface of the major wall 11 of the flask 10. The cell culture device 1 is then held within the recess of an automated storage system. When the cell population has exhausted the cell culture medium in the smaller flask 10, or when the population is sufficiently large that the area in which the cells are confined is sufficiently small to inhibit further population growth, the cells may be transferred into the larger flask 100 to continue growing. The transfer can be effected either by using a pipette 23 or by securing the cap 117 on the larger flask 100 and then inverting the cell culture device 1 to cause the cells to detach from the growing surface and move into the larger flask 100. The cell culture device 1 is then returned to the automated cell storage device to allow the cells to continue to grow. Once the population density in the larger flask 100 is sufficiently high the cells can be removed from the flask 100. During this operation it is preferable that none of the debris from the original incubation in the smaller flask 10 contaminates the contents of the larger flask. This operation is therefore preferably performed using a pipette 23 although it is possible, in some examples of the present invention, to rely on the tapering of the neck portion of the small flask to contain such debris.

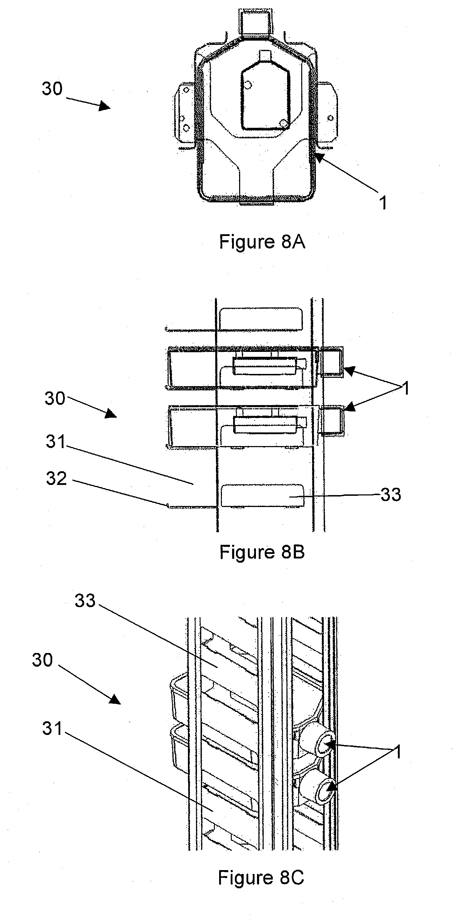

Part of an automated cell storage device 30 is shown in Figure 8. The storage unit 30 has a large number of recesses 31 each of which can hold a cell culture device 1 or a standard flask. The cell culture device 1 or flask is held in position by a number of retaining members 32, 33 that interface with the base 115 and minor walls 113, 144 of the cell culture device 1. Depending on the type of system the temperature of the cells can be appropriately controlled to optimise the growth or to maintain the integrity of the culture for long term storage as appropriate.

## Claims

1. A cell culture device comprising:
a flask within which, in use, cells are cultured;
a structure connected to the flask and constructed to enable the flask to be held, in use, within a recess of a cell culture system;
wherein the volume of the envelope defined by the structure and the flask is greater than the volume of the flask.

2. A cell culture device according to claim 1, wherein the flask is held within the envelope defined by the structure.

3. A cell culture device according to claim 1 or claim 2, wherein the structure comprises a cuboid framework.

4. A cell culture device according to any of claims 1 to 3, wherein the structure comprises a larger flask than the smaller flask in which the cells are, in use, cultured.

5. A cell culture device according to any of claims 1 to 4, wherein the larger flask has an opening that is arranged to as to permit access to the contents of the smaller flask.

6. A cell culture device according to any of claims 1 to 5, wherein the smaller flask further comprises a closure member.

7. A cell culture device according to any of claims 1 to 6, further comprising at least one spacing member arranged so as to separate the flask from the structure.

8. A cell culture device according to any of claims 4 to 6, wherein the smaller flask and larger flask each comprise at least one shared wall.

9. A cell culture device according to any of the preceding claims wherein the envelope of the structure is adapted to conform to the recess.

10. An automated storage system comprising a plurality of cell culture devices according to any of claims 1 to 8.
